## Europäisches Patentamt

## European Patent Office
## Office européen des brevets

(11) Veröffentlichungsnummer: **0 024 442**
**A1**

(12) # EUROPÄISCHE PATENTANMELDUNG

(21) Anmeldenummer: **79103084.4**

(22) Anmeldetag: **22.08.79**

(51) Int. Cl.³: **A 61 F 1/03**

(43) Veröffentlichungstag der Anmeldung:
**11.03.81 Patentblatt 81/10**

(84) Benannte Vertragsstaaten:
**AT BE CH DE FR GB IT LU NL SE**

(71) Anmelder: **Rosenthal Technik AG**
**Postfach 1508**
**D-8672 Selb/Bayern(DE)**

(72) Erfinder: **Zeibig, Anton, Dr.**
**Bergstrasse 18**
**D-8561 Ottensoos(DE)**

(54) **Verbund-Endoprothese aus einem Metallschaft und einem keramischen Gelenkteil.**

(57) Verbund-Endoprothese, insbesondere Gelenk-Endoprothese, bestehend aus einem keramischen Gelenkteil (1) und einer konischen oder zylindrischen Bohrung (8), wobei das Gelenkteil (1) mit seiner inneren Domfläche (2) auf der Stirnfläche (3) des Metallzapfens (4) aufsitzt und der Halt des Gelenkteils (1) durch elastische und plastische deformierbare Tragrippen (5) am Zapfen (4) des Metallschaftes (6) erfolgt. Die hochfeste Verbindung zwischen Gelenkteil (1) und Metall-Zapfen (4) erfolgt durch Preßsitz. Solche Verbund-Endoprothesen sind zur Implantation im Bereich der Humanmedizin für die Gelenke der Hüfte, Schulter und des Knies geeignet.

Fig. 1a

EP 0 024 442 A1

0024442

ROSENTHAL TECHNIK AG

Selb, den 9. August 79
Re/li

RT.P. 1335

---

## Verbund-Endoprothese aus einem Metallschaft und einem keramischen Gelenkteil

---

Die Erfindung betrifft eine Verbund-Endoprothese, insbesondere Gelenk-Endoprothese, bestehend aus einem metallischen Schaft und einem keramischen Implantatsteil mit einer konischen oder zylindrischen Bohrung, wobei die hochfeste Verbindung zwischen den beiden Endoprothesen-Teilen durch Preßsitz erfolgt. Solche Gelenk-Endoprothesen sind besonders zur Implantation im Bereich der Humanmedizin für die Gelenke der Hüfte, Schulter und des Knies geeignet.

Es ist davon auszugehen, daß mechanisch hochfeste Verbindungen durch Kraftschluß in der Technik bekannt sind und vielfach angewendet werden, z. B. Schrumpfsitz zwischen Nabe und Welle, Befestigung einer Schraube mittels Kunststoffdübel und bei der Stieber-Rollkupplung. Es kommt dabei im wesentlichen darauf an, wie der Kraftschluß erzielt wird. Beim Schrumpfsitz wird er durch die Ausnutzung der thermischen Dehnung erreicht; beim Kunststoffdübel wird der Effekt durch die plastische Deformation des Kunststoffeinsatzes und bei der Rollkupplung durch die elastische Deformation einer Metallhülse mittels einer Rollageranordnung bewirkt.

Bekannt auf dem Gebiet der Endoprothesen sind ebenfalls solche kraftschlüssigen Verbindungen wie z. B. aus der DE-OS 25 48 077 hervorgeht. Bei der in dieser Druckschrift offenbarten Gelenk-Endoprothese erfolgt die Verbindung zwischen dem Metallschaft und dem Keramikkopf in der Weise, daß der Keramikkopf mit seiner Bohrung unmittelbar auf den Zapfen des Metallschaftes aufgesetzt wird. Weiterhin wird in der genannten Druckschrift vorgeschlagen, um einen sicheren Reibschluß zu erhalten und andererseits die Gefahr der Sprengung des Keramikkopfes durch die Erhitzung der Endoprothese bei der Sterilisation zu mindern, den Zapfen bzw. die Bohrung im Keramikkopf mit einem Rillenprofil zu versehen, um dadurch Deformationsräume zwischen dem Metall-Zapfen und dem keramischen Gelenkteil zu schaffen. Es hat sich jedoch gezeigt, daß diese Verbindungstechnik mit selbsthemmenden Gewindezapfen und einem Keramikkopf mit Rillenprofil in der Bohrung eine sehr hohe Sprengwirkung auf das Gelenkteil ausübt. Außerdem muß der Sitz des Gelenkkopfes in mehreren Arbeitsgängen ausgearbeitet und mit einer außerordentlichen Präzision ausgeführt werden, weil sich sonst eine fehlerhafte Lastverteilung ergibt, die entweder beim Montieren des Schaftes oder beim Gebrauch der Prothese den Bruch des Gelenkkopfes aus keramischem Material hervorruft.

Die Aufgabe der Erfindung besteht darin, bei geringem Herstellungsaufwand und einem relativ niedrigen Präzisionsgrad eine einfache und zuverlässig feste Verbindung der beiden Endoprothesenteile zu erhalten, bei der auch die zwischen dem Zapfen des Schaftes und dem keramischen Gelenkteil notwendige korrekte Lastverteilung erzielt wird und keine mechanische Überbeanspruchung des Gelenkteils entsteht.

Zur Lösung dieser Aufgabe sind bei der Erfindung die im kennzeichnenden Teil des ersten Anspruches angegebenen Merkmale vorgesehen. Durch das direkte Aufsitzen der inneren Domfläche des keramischen Gelenkteils auf die Stirnfläche des Metall-Zapfens wird erreicht, daß die Punkte der äußeren Krafteinleitung in den kleinstmöglichen Abstand zu den Punkten der inneren Kraftübertragung von der Domfläche auf die Stirnfläche des Metallschaftes kommen oder mit anderen Worten, daß der mechanische Kraftfluß auf kürzestem Wege verläuft. Das heißt, die zwischen dem keramischen Gelenkteil und dem Befestigungszapfen des Metallschaftes auftretenden Kippmomente sind weitgehend reduziert. Sofern es jedoch die speziellen konstruktiven und festigkeitsmäßigen Anforderungen verlangen oder zulassen, kann auch der Unterstützungsansatz am Fuße des keramischen Gelenkteils angewendet werden, ohne daß dadurch die übrigen Vorteile der Verbindungslösung verloren gehen.

Durch den Wegfall des Unterstützungsansatzes am Fuße des keramischen Gelenkteils kann in sehr vorteilhafter Weise der räumliche Winkel der Beweglichkeit für die Artikulation des Gelenkteils z. B. in einer kugeligen Halbschale so einer Pfanne aus Kunststoff oder Keramik gewährleistet werden. Auch kann man das keramische Gelenkteil direkt in die natürliche Pfanne des Hüftgelenkes einsetzen. Eine zweckmäßige Ausgestaltung des Zapfens besteht darin, daß die Tragrippen geradlinig und ringförmig auf der Oberfläche des Zapfens ausgebildet sind. Eine Variation dieser Tragrippen besteht auch darin, daß an der ringförmigen Außenfläche der Tragrippen Schlitze eingefügt werden, wodurch die federnde Wirkung erhöht werden kann, was auf die größeren Federwege der Federlamelle zurückzuführen ist. Durch die elastische und plastisch deformierbaren Tragrippen des Metall-Zapfens wird erreicht, daß ohne Verwendung einer Präzisionsbohrung die Verbindung zwischen dem keramischen Gelenkteil und dem Metall-Zapfen in einfacher Weise hergestellt werden kann. Wichtig ist dabei, daß der Metall-Zapfen auf das keramische Gelenkteil keine unkontrollierte Sprengwirkung mehr ausüben kann, wie das zum Beispiel beim Massivkonus der Fall ist. Durch die Ausnutzung der plastischen und elastischen Deformation der Tragrippen und dem damit erreichbaren Deformationsweg kann zum Festsitz auf dem Metall-Zapfen die Schleifbearbeitung der Innenbohrung des keramischen Gelenkteils wegfallen.

Andererseits kann der Zapfen als Hohlkonus ausgebildet sein, womit die Anpassung der Geometrien von Zapfen und Bohrung des Gelenkteils durch die größere Deformierbarkeit des Metall-Zapfens verbessert wird.

Für bestimmte Belastungsarten wie z. B. hoher Druck in axialer Richtung, kann es von Vorteil sein, den Dom der Bohrung des keramischen Gelenkteils halbkugelförmig oder flach zu gestalten und das Ende des Metallzapfens entsprechend zu verformen. Der notwendige Toleranzausgleich kann dabei durch das Anbringen kurzer Deformationsstrecken auf der Stirnfläche des Metall-Zapfens bzw. das Einbringen einer dünnen Deformationsschicht erreicht werden. Diese Schicht kann dabei in fester Form oder als härtender anorganischer und/oder organischer Kitt eingebracht werden.

Die Rotationssymmetrie der Bohrung des keramischen Gelenkteils kann auf ihrer Länge ganz oder teilweise z. B. durch ein- oder mehrseitige Abflachung oder ganz allgemein durch eine vom Krümmungsradius verschiedene Linienführung verlassen werden, um einen Formschluß mit dem Metallzapfen zwecks Rotationssicherung zu bewirken.

Diese und andere Vorteile, die aus der nachfolgenden Beschreibung näher hervorgehen, vereinigt in sich die erfindungsgemäße Endoprothese aus einem Metallschaft und einem im wesentlichen kugeligen Gelenkteil aus keramischem Werkstoff, der über das zapfenförmige Endstück des Schaftes fest mit diesem verbunden ist.

Die Erfindung und deren weitere Ausgestaltungsmöglichkeiten wird anhand von Ausführungsbeispielen, die schematisch in den Fig. 1 - 9 wiedergegeben sind, näher erläutert. Es zeigen im einzelnen:

Fig. 1a + b: Verbund-Endoprothese mit teilweisem Schnitt des keramischen Gelenkteils,

Fig. 2 : Seitenansicht des Metall-Zapfens, des keramischen Gelenkteils und einem pfannenartigen Teil,

Fig. 3 : Ausbildung der Tragrippen in Form von Federlamellen,

Fig. 4 : Befestigungszapfen mit Axialbohrung von der Stirnfläche ausgehend und Darstellung der Variierbarkeit der Anzahl der Tragrippen,

Fig. 5 : Dom des keramischen Gelenkteils mit halbkugelförmiger Wölbung,

Fig. 6     : Flacher Dom des kugeligen Gelenkteils und
             Innenbohrung mit umlaufender Arretierschulter,

Fig. 7     : Befestigungszapfen der Metallschaftprothese
             mit deformierbarem Oberflächenprofil,

Fig. 8     : Deformationselement zwischen dem flachen Dom des
             keramischen Gelenkteils und der Stirnfläche des
             Metall-Zapfens,

Fig. 9     : Abflachung der zylindrischen oder konischen
             Bohrung des keramischen Gelenkteils in Aufsicht.

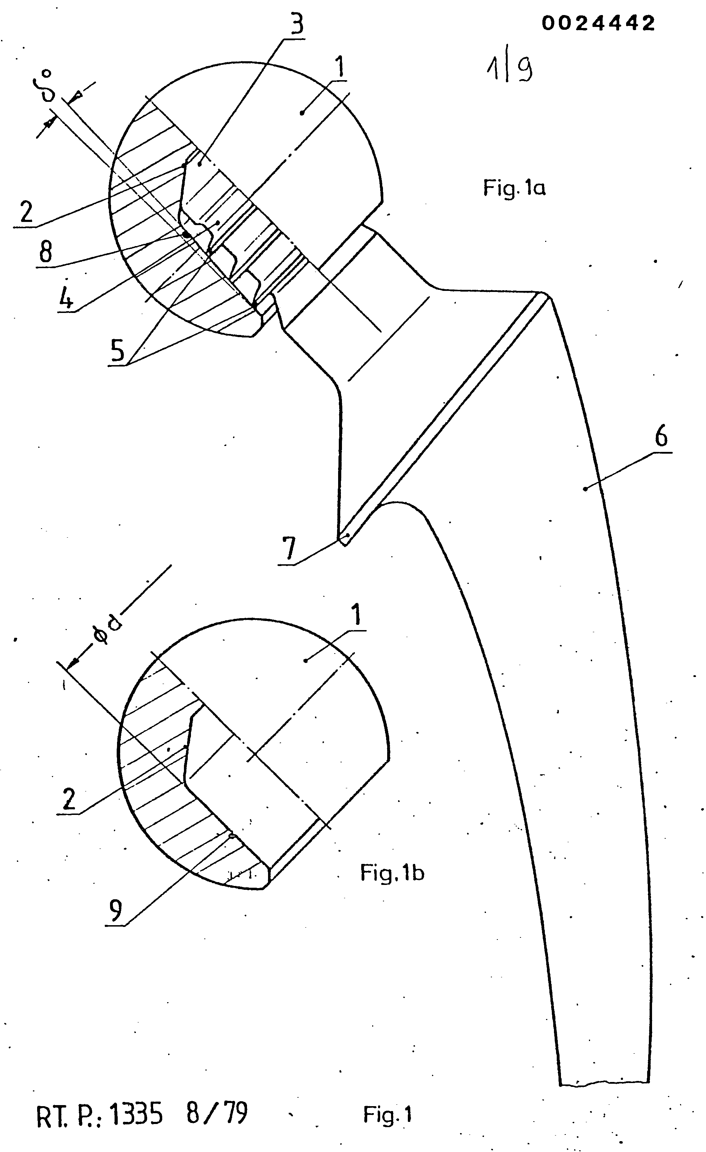

Die erfindungsgemäße Verbund-Endoprothese aus Fig. 1a + b besteht aus einem keramischen Gelenkteil 1, das mit seiner inneren Domfläche 2 auf der Stirnfläche 3 des Metall-Zapfens 4 direkt aufsitzt. Der Metall-Zapfen 4 des Schaftes 6 besteht aus einer Chrom-Kobalt-Molybdän-Legierung, Chrom-Nickel-Stahllegierung oder aus einer Titanlegierung und ist konisch oder zylindrisch ausgebildet und weist mehrere elastische und plastisch deformierbare Tragrippen 5 auf. Die Tragrippen selbst sind 0,3 - 2 mm dick, 1 - 6 mm tief und weisen einen Abstand von 1 - 7 mm in Längsrichtung des Metall-Zapfens 4 auf. Die Schulter 7 des Metallschaftes 6 dient in bekannter Weise der festen Auflage des Metall-Implantates auf dem proximalen Ende des Knochens, wie das z. B. im Falle eines für ein künstliches Hüftgelenk verwendeten Metall-Implantats der Fall sein muß.

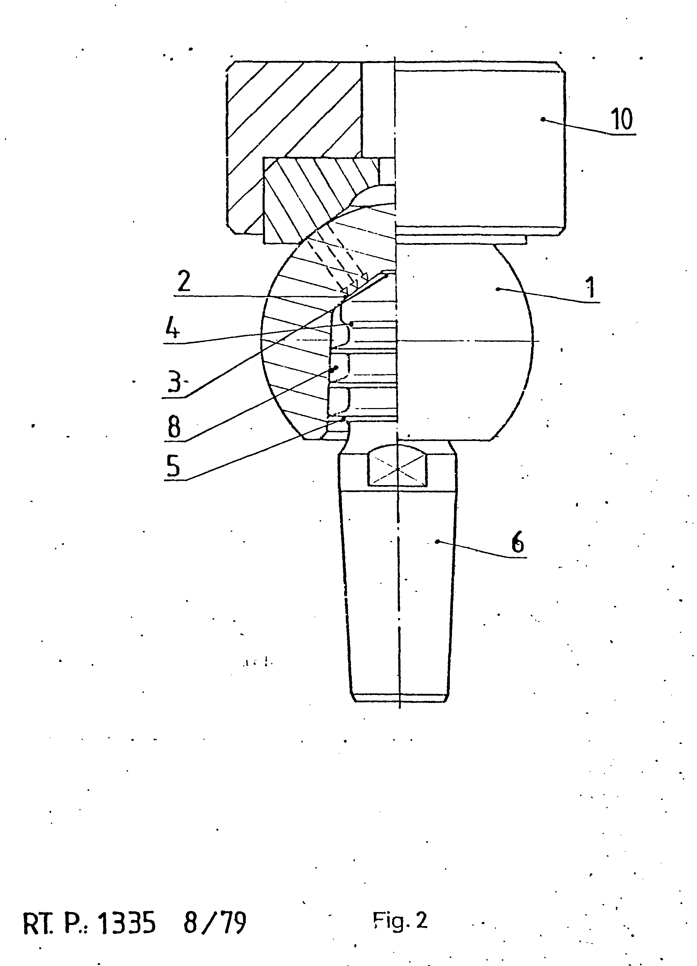

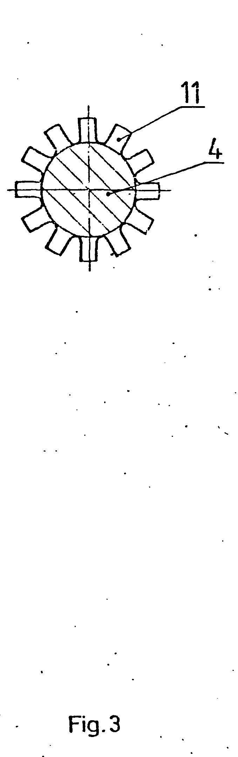

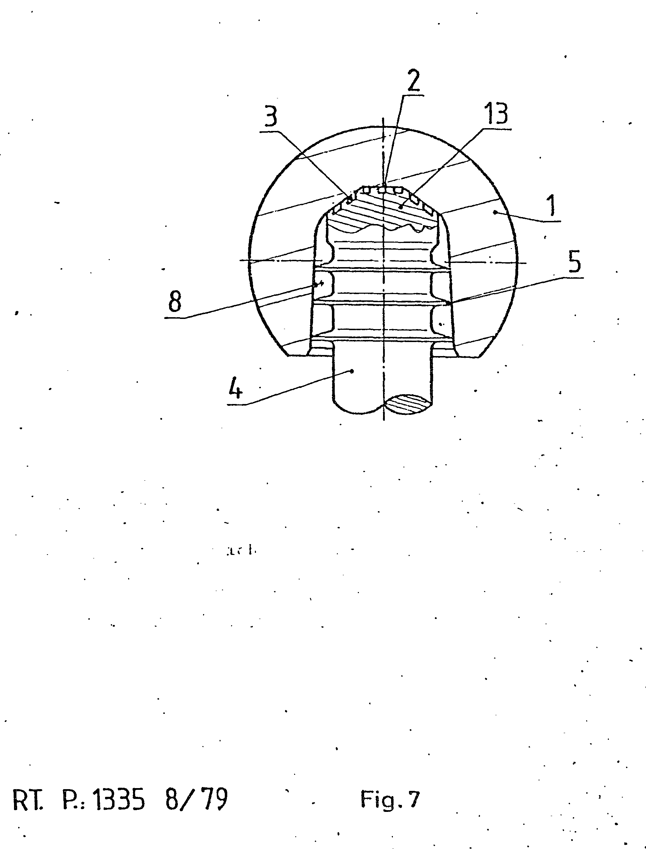

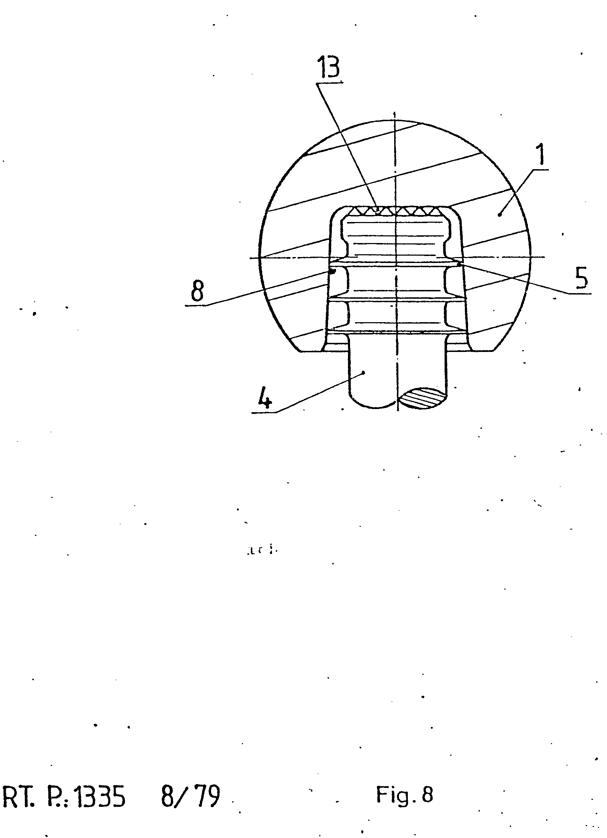

Aus Fig. 2 geht hervor, daß der Preßsitz zwischen Gelenkteil 1 mit konischer oder zylindrischer Bohrung 8, 9 und Metall-Zapfen 4 mit einer vorgegebenen auf den Scheitelpunkt des Gelenkteils 1 wirkenden Kraft erzeugt wird. Durch diese Aufpreßkraft entsteht eine elastische und plastische Deformation der Tragrippen 5 wie in Fig. 3 dargestellt ist und auch als Federlamelle 11 ausgebildet sein kann. Die von den Tragrippen ausgehenden Festhaltekräfte verteilen sich gleichmäßig längs ihrer Umfanglinie und übertragen sich somit auch gleichmäßig auf das Gelenkteil 1. Durch die Bemessung der Tragrippen 5 wird sichergestellt, daß die Festhaltekräfte insgesamt nicht größer sind als der zum sicheren Beherrschen des bei einer auf die Prothese wirkenden Schräglast entstehenden Kippmoments notwendige Betrag. Dabei wird die Axialkomponente der Schräglast von der Pfanne 10 auf kurzem Wege vom Dom des Gelenkteils 1 auf die Stirnfläche 3 des Metallzapfens 4 übertragen. Diese Stirnfläche 3 kann auch als Deformationsfläche 13 ausgebildet sein, wie aus Fig. 7 und 8 hervorgeht.

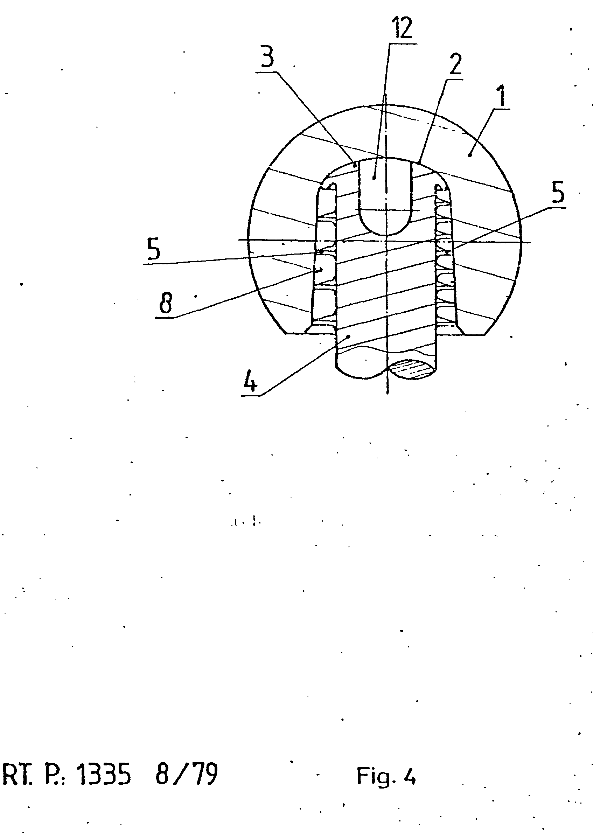

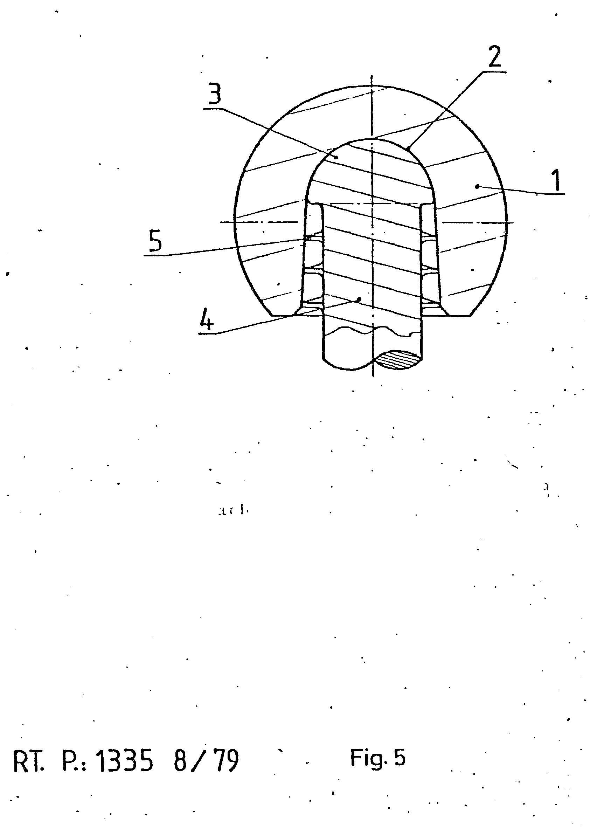

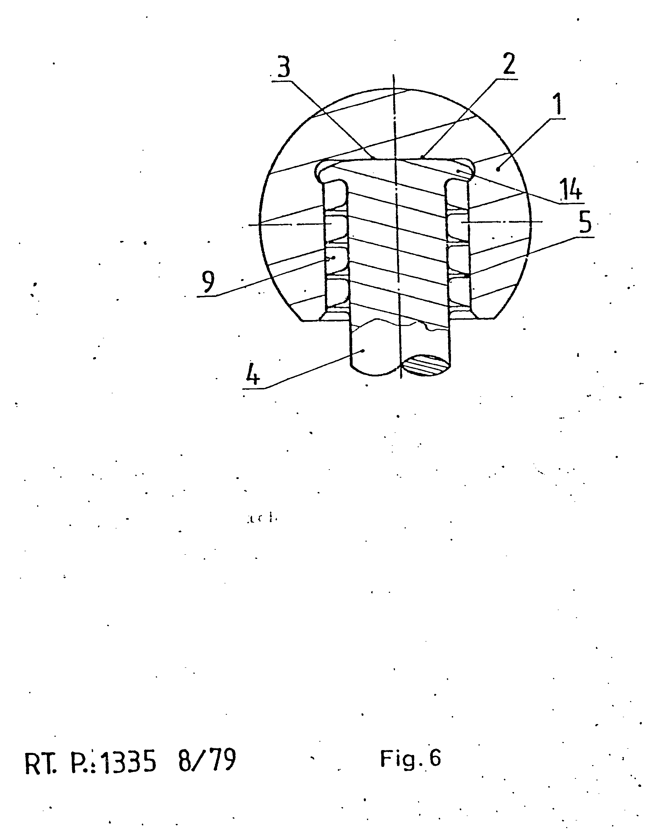

Variationen der inneren Domfläche 2 des keramischen Gelenkteils als auch der Stirnfläche 3 des Metall-Zapfens 4 gehen aus der Fig. 4, 5 und 6 hervor. Bei Fig. 4 ist insbesondere auf den Hohlkonus 12 hinzuweisen, der noch einmal zusätzlich Deformationskräfte aufnehmen kann. Bei der Fig. 5 ist sowohl die Domfläche 2 des keramischen Gelenkteils 1 als auch die Stirnfläche 3 des Metall-Zapfens 4 halbkugelförmig ausgestaltet. Aus Fig. 6 geht nicht nur hervor, daß man die innere Domfläche und entsprechend auch die Stirnfläche 3 des Metallzapfens 4 flach ausbilden kann, sondern auch noch zusätzlich eine Arretierschulter 14 anbringen kann, die insbesondere bei einer zylindrischen Bohrung 9 ein Abziehen des keramischen Gelenkteils 1 verhindert.

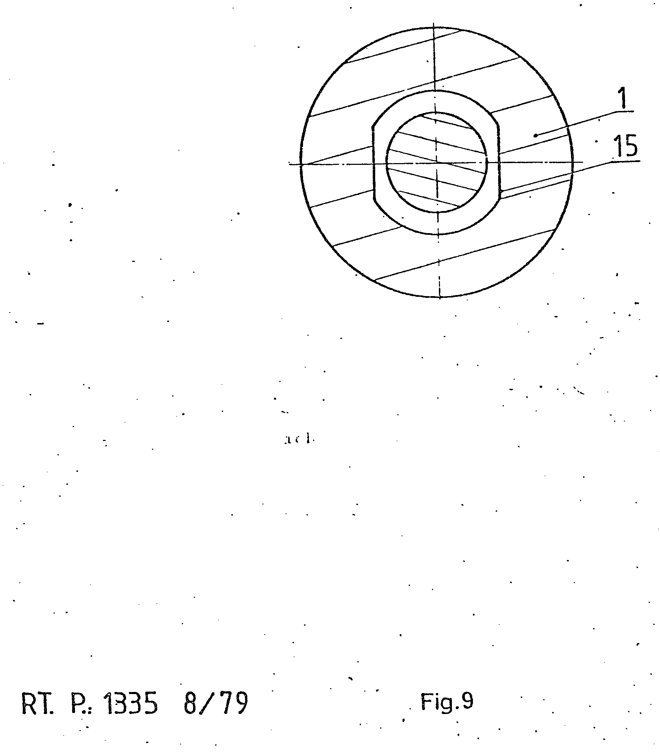

Um eine zusätzliche Sicherung gegen Rotation des Gelenkteils 1 zu schaffen, ist es sinnvoll, die Bohrung 8, 9 mit Abflachungen 15 zu versehen, wie aus der Fig. 9 hervorgeht.

Durch die erfindungsgemäßen Merkmale der Gelenkendoprothese wird sichergestellt, daß die hohe Druckfestigkeit des für den Gelenkteil verwendeten keramischen Materials wirkungsvoll zum Einsatz gebracht wird. Bekanntlich ist bei den keramischen Werkstoffen die Druckfestigkeit mindestens fünfmal so hoch wie die Zug- und die Biegefestigkeit. Bei dem für das Gelenkteil verwendeten biokeramischen Werkstoffes aus Aluminiumoxid beträgt die Druckfestigkeit mindestens 25.000 $dN/cm^2$, wo hingegen die Zugefestigkeit mindestens 3.000 $dN/cm^2$ und die Biegefestigkeit mindestens 4.000 $dN/cm^2$ betragen. Für ein Ausführungsbeispiel eines kugelförmigen Gelenkteils mit 32 mm Außendurchmesser und 16 mm Innendurch- messer ergab die Festigkeitsberechnung einen Wert für die Axiallast von 6.000 dN, der in den praktischen Belastungs- prüfungen voll bestätigt wurde.

0024442

- 9 -

Stückliste

| | | |
|---|---|---|
| | Gelenkteil | 1 |
| | Domfläche | 2 |
| | Stirnfläche | 3 |
| | Metall-Zapfen | 4 |
| | Tragrippen | 5 |
| | Metallschaft | 6 |
| | Schulter | 7 |
| | Konische Bohrung | 8 |
| | Zylinderförmige Bohrung | 9 |
| | Pfanne | 10 |
| | Federlamelle | 11 |
| | Hohlkonus | 12 |
| | Deformationsfläche | 13 |
| | Arretierschulter | 14 |
| | Abflachungen | 15 |

P a t e n t a n s p r ü c h e

1. Verbund-Endoprothese, insbesondere Gelenk-Endoprothese, bestehend aus einem metallischen Schaft und
einem keramischen Implantatsteil mit einer konischen oder
zylinderförmigen Ausnehmung, wobei die hochfeste Verbindung
zwischen den beiden Endoprothesenteilen durch Preßsitz erfolgt, d a d u r c h   g e k e n n z e i c h n e t, daß das
Gelenkteil (1) mit seiner inneren Domfläche (2) auf der
Stirnfläche (3) des Metallzapfens (4) aufsitzt und der
Halt des Gelenkteils (1) durch elastische und plastisch
deformierbare Tragrippen (5) am Zapfen (4) des Metallschaftes (6) erfolgt.

2. Verbund- Endoprothese nach Anspruch 1, d a d u r c h
g e k e n n z e i c h n e t, daß die Tragrippen (5) ringförmig auf der Oberfläche des Metall-Zapfens (4) sitzen.

3. Verbund-Endoprothese nach Anspruch 1 und 2, d a d u r c h
g e k e n n z e i c h n e t, daß die Tragrippen (5) geschlitzt
als Federlamelle (11) ausgeführt sind.

4. Verbund-Endoprothese nach Anspruch 1 - 3, dadurch gekennzeichnet, daß die Tragrippen (5) selbst 0,3 - 2 mm dick bzw. 1 - 6 mm tief sind und einen Abstand von 1 - 7 mm in Längsrichtung des Metall-Zapfens (4) aufweisen.

5. Verbund-Endoprothese nach Anspruch 1 - 4, dadurch gekennzeichnet, daß der Metall-Zapfen (4) als Hohlkonus (12) ausgebildet ist.

6. Verbund-Endoprothese nach Anspruch 1 - 4, dadurch gekennzeichnet, daß die Domfläche (2) der Bohrung (8, 9) des keramischen Gelenkteils (1) und die Stirnfläche (3) des Metallzapfens (4) halbkugelförmig gestaltet sind.

7. Verbund-Endoprothese nach Anspruch 1 - 4, dadurch gekennzeichnet, daß die Domfläche (2) der Bohrung (8,9) des keramischen Gelenkteils (1) und die Stirnfläche des Metall-Zapfens (4) flach ausgeführt sind.

8. Verbund-Endoprothese nach Anspruch 1 - 7, dadurch gekennzeichnet, daß die Stirnfläche (3) des Metallzapfens (4) deformierbar ist.

9. Verbund-Endoprothese nach Anspruch 1 - 8, dadurch gekennzeichnet, daß die Bohrung (8, 9) des keramischen Gelenkteils (1) eine Arretierschulter (14) aufweist.

10. Verbund-Endoprothese nach Anspruch 1 - 8, dadurch gekennzeichnet, daß die zylindrische oder konische Bohrung (8, 9) des keramischen Gelenkteils (1) Abflachungen aufweist.

Fig. 1a

Fig. 1b

Fig. 1

RT. P.: 1335  8/79

Fig. 2

Fig. 3

RT. P.: 1335 8/79

Fig. 4

RT. P.: 1335   8/79

Fig. 5

6/9

RT. P.: 1335  8/79          Fig. 6

7/9

RT. P.: 1335 8/79          Fig. 7

Fig. 8

Fig.9

RT. P. 1335   8/79

0024442

**Europäisches Patentamt**

# EUROPÄISCHER RECHERCHENBERICHT

Nummer der Anmeldung

EP 79 10 3084

| | EINSCHLÄGIGE DOKUMENTE | | KLASSIFIKATION DER ANMELDUNG (Int.Cl.³) |
|---|---|---|---|
| Kategorie | Kennzeichnung des Dokuments mit Angabe, soweit erforderlich, der maßgeblichen Teile | betrifft Anspruch | A 61 F 1/03 |
| | DE - B1 - 2 548 591 (GEBR. SULZER)<br>* Ansprüche 1, 5; Spalte 2, Zeilen 26 bis 33 *<br>-- | 1,2,<br>5,7 | |
| A | DE - A1 - 2 742 098 (ROSENTHAL TECHNIK)<br>* Anspruch 1 *<br>-- | 1 | |
| A | DE - A1 - 2 340 734 (FELDMÜHLE)<br>* Fig. 1 *<br>-- | 1 | **RECHERCHIERTE SACHGEBIETE (Int. Cl.³)** |
| D | DE - A1 - 2 548 077 (FELDMÜHLE)<br>* Ansprüche 1, 3, 4 *<br>---- | 1,2 | A 61 F 1/00 |

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Berlin | 16-04-1980 | KANAL |

EPA form 1503.1 06.78